# EUROPEAN PATENT APPLICATION

(11) **EP 4 349 198 A1**
(43) Date of publication of application: **10.04.2024**
(21) Application number: 21943088.1
(22) Date of filing: 28.05.2021
(51) Int. Cl.: A24F 40/65

(54) **INFORMATION PROCESSING METHOD, INFORMATION PROCESSING TERMINAL, AND INFORMATION PROCESSING SYSTEM**

(71) Applicant: Japan Tobacco, Inc., Tokyo, 105-6927 (JP)
(72) Inventor: OSAWA, Kosuke, Tokyo 130-8603 (JP)
(74) Representative: Hoffmann Eitle
(86) International application number: PCT/JP2021/020381
(87) International publication number: WO 2022/249443

(57) **Abstract**

Provided is an information processing method that enables flexible setting of the operations of an inhalation device from a remote location using a user terminal and that provides usability for preventing the inhalation device from implementing an operation setting not intended by a user due to an erroneous manipulation by the user. This information processing method includes: a step for accepting a first setting request relating to setting of the operations of an inhalation device; a step for transmitting the first setting request, which is addressed to the inhalation device, to a server device; a step for causing a displayed status relating to the inhalation device to transition to "standby" in accordance with the transmission of the first setting request; a step for receiving a completion verification, which indicates that the inhalation device has completed the operation setting, from the inhalation device via the server device; and a step for causing the displayed status to transition from "standby" to "finished" in accordance with the completion verification.

## Description

### TECHNICAL FIELD

The present invention relates to an information processing method, an information processing terminal, and an information processing system.

### BACKGROUND ART

An inhaler used by a user for inhaling a flavor has been known widely. Such inhalers include aerosol generators and flavor inhalers. An aerosol generator is an electronic device product used for inhaling generated aerosol. Although there is no intention to limit the scope, the aerosol generators include, for example, electronic cigarettes, heated tobacco products, and nebulizers. In this regard, the aerosol generators include a product which is so-called "RRP (Reduced-Risk Product)" which may reduce a health risk of a user relating to smoking.

In the inhales such as those explained above, there are some inhalers which cooperate, via communication, with user terminals such as smartphones. For example, Patent Literature 1 discloses a technique for sending data to an external device when an aerosol generator has detected dropping thereof or an impact received thereby. Patent Literature 2 discloses a technique for allowing communication between a vaporizer and a mobile communication device, and making the vaporizer operate in accordance with a parameter that is determined according to an input to the mobile communication device. Each of Patent Literatures 3 and 4 discloses a technique for allowing communication between an inhaler and an external computing device, and activating the inhaler (for example, changing the state thereof from a lock state to an unlock state) via manipulation of the external computing device by a user (Patent Literature 5 also discloses a technique similar to the above technique).

### CITATION LIST

### PATENT LITERATURE

PTL 1: PCT pamphlet No. WO 2019/186468
PTL 2: PCT pamphlet No. WO 2019/104227
PTL 3: Japanese Patent Application Public Disclosure No. 2018-522649
PTL 4: Japanese Patent Application Public Disclosure No. 2018-507497
PTL 5: Japanese Patent Application Public Disclosure No. 2018-509139

### SUMMARY OF INVENTION

### TECHNICAL PROBLEM

It is desired to realize an information processing system which makes it possible to send/receive various kinds of data to allow, in a flexible manner, remote setting of operation of an inhaler by using a user terminal. On the other hand, a user is required to manipulate an application in a user terminal in an appropriate manner, for preventing applying of unintended operation settings to an inhaler.

Especially, in the case wherein a user does not have an inhaler at hand and is not able to confirm its operation state directly, the user is required to grasp the state of the setting of operation of the inhaler precisely by using an application in a user terminal, and avoid applying erroneous manipulation to the user terminal. The above matter is especially applicable to a case wherein communication between an inhaler and a user terminal is asynchronous communication involving time lag. Further, since an inhaler often comprises various kinds of components for heating, it is required to consider safety with respect to the above components if setting of operation of the inhaler is performed by a user remotely.

Accordingly, an object of the present disclosure is to provide a construction that allows to perform setting of operation of an inhaler remotely in a flexible manner by using a user terminal. Further, an object of the present disclosure is to provide usability that prevents a user from performing erroneous manipulation to thereby prevent applying of unintentional operation settings to an inhaler.

### SOLUTION TO PROBLEM

For achieving the above objects, an information processing method is provided according to an aspect of the present disclosure, wherein the information processing method comprises steps for: accepting a first setting request relating to operation setting of the inhaler; transmitting the first setting request, that is directed to the inhaler, to a server; changing, in response to transmission of the first setting request, a display status relating to the inhaler to that representing a waiting-for-reflection state; receiving, from the inhaler via the server, a completion confirmation that represents completion of the operation setting in the inhaler; and changing, in response to the completion confirmation, the display status from that representing the waiting-for-reflection state to that representing a reflection-completed state.

It may further comprises a step for disallowing accepting of a second setting request relating to operation setting of the inhaler, during a period of time when the display status is that representing the waiting-for-reflection state.

It may further comprise a step for allowing accepting of the second setting request, in response to completion of changing of the display status from that representing the waiting-for-reflection state to that representing the reflection-completed state.

A predetermined restriction may be related to communication with the inhaler, in a wireless network to which the inhaler is connected; the above method may further comprise a step for receiving, from the server after the first setting request is transmitted to the server, restriction information determined in the server; and the restriction information may comprise waiting time until the time when the predetermined restriction is removed.

The operation setting of the inhaler may be related to at least one of a function relating to a profile of heating operation in the inhaler, a function of locking operation of the inhaler, and a function of displaying of information inputted by a user.

The operation setting may be related to plural functions that the inhaler has, and each of the plural functions may be related to the display status.

The plural functions may comprise a first function and a second function; the first setting request may be related to the first function; and, in response to transmission of the first setting request, the display statuses relating to the inhaler, that relate to the first function and the second function, may be changed to represent the waiting-for-reflection states, respectively.

Further, an information processing terminal is provided according to an aspect of the present disclosure, wherein the information processing terminal comprises: a reception unit for accepting a first setting request relating to operation setting of an inhaler; a transmitter for transmitting the first setting request, that is directed to the inhaler, to a server; a receiver for receiving, from the inhaler via the server, a completion confirmation that represents completion of the operation setting in the inhaler; and a status transition unit for changing, in response to transmission of the first setting request, a display status relating to the inhaler to that representing a waiting-for-reflection state, and changing, in response to a completion confirmation from the inhaler, the display status from that representing the waiting-for-reflection state to that representing a reflection-completed state.

It may further comprise a reception controller which disallows accepting of a second setting request relating to operation setting of the inhaler, during a period of time when the display status is that representing the waiting-for-reflection state.

The reception controller may further be constructed to allow accepting of the second setting request, in response to completion of changing of the display status from that representing the waiting-for-reflection state to that representing the reflection-completed state.

A predetermined restriction may be related to communication with the inhaler, in a wireless network to which the inhaler is connected; it may be constructed in such a manner that the receiver receives, from the server after the first setting request is transmitted to the server from the transmitter, restriction information determined in the server; and the restriction information may comprise waiting time until the time when the predetermined restriction is removed.

Further, an information processing system comprising an information processing terminal, a server, and an inhaler is provided according to an aspect of the present disclosure, wherein: the information processing terminal comprises a reception unit for accepting a setting request relating to operation setting of the inhaler, and a first communicator for transmitting the setting request, that is directed to the inhaler, to a serve, and receiving, from the server, a completion confirmation that represents completion of the operation setting in the inhaler; the server comprises a second communicator for transmitting, to the inhaler via an external server, the setting request received from the information processing terminal, and transmitting, to the information processing terminal, the completion confirmation received from the inhaler via the external server, and a communication manager for managing, based on a predetermined restriction defined with respect to communication between the external server and the inhaler, transmission of the setting request by the second communicator; and the inhaler comprises a third communicator for transmitting, to the external server, the completion confirmation relating to the setting request received from the server via the external server, and a controller for performing, in response to the setting request, the operation setting in the inhaler.

The information processing terminal may further comprise a status transition unit for changing, in response to transmission of the setting request, a display status relating to the inhaler to that representing a waiting-for-reflection state, and changing, in response to the completion confirmation, the display status from that representing the waiting-for-reflection state to that representing a reflection-completed state; and the display status may be displayed by the information processing terminal.

The communication manager in the server may be constructed to judge, in response to reception of the setting request by the second communicator, whether the communication is restricted, and hold transmission of the setting request to the inhaler, if the communication is restricted.

The communication manager in the server may be constructed to determine restriction information that is based on the predetermined restriction; the second communicator in the server may be constructed to transmit the restriction information to the information processing terminal; and the restriction information may comprise waiting time until the time when the restriction relating to the communication is removed.

The waiting time may be determined based at least on history of past waiting time that relates to the user and has been recorded in the server.

The predetermined restriction relating to the communication may comprise a restriction such that transmission of the setting request to the inhale is allowed to be performed only a predetermined number of times within a predetermined period of time.

The predetermined restriction relating to the communication may comprise a restriction such that transmission of the setting request to the inhaler is allowed to be performed only within a predetermined allowed period of time starting at each predetermined time interval.

The third communicator in the inhaler may further be constructed to periodically transmit a communication request to the external server, and receive the setting request as a response to the communication request.

The wireless network, to which the inhaler is connected, may conform to an LPWA (Low Power Wide Area) wireless communication standard.

### ADVANTAGEOUS EFFECTS OF INVENTION

As explained above, according to the present disclosure, it becomes possible to provide a user with usability that prevents the user from performing erroneous manipulation with respect to operation of an inhaler, when the user performs setting of operation of the inhaler remotely by using a user terminal.

### BRIEF DESCRIPTION OF DRAWINGS

Fig. 1 is a figure which schematically shows a general construction of an information processing system according to a present embodiment.
Fig. 2A is a figure which schematically shows a construction example of an aerosol generator.
Fig. 2B is a figure which schematically shows a different construction example of an aerosol generator.
Fig. 3 is a figure which schematically shows a hardware construction example of a user terminal.
Fig. 4 is a figure which schematically shows a hardware construction example of a server.
Fig. 5 is a figure which schematically shows an example of a functional construction of an information processing system according to a present embodiment.
Fig. 6 is a schematic flow chart of an example of processing in an information processing system according to a present embodiment.
Fig. 7 is an example detailed flow chart of processing steps included in some processing steps shown in Fig. 6.
Fig. 8A is a figure which shows an example of a screen image displayed by a user terminal.
Fig. 8B is a figure which shows a different example of a screen image displayed by a user terminal.

### DESCRIPTION OF EMBODIMENTS

In the following description, information processing methods, information processing terminals, and information processing systems according to embodiments of the present disclosure will be explained with reference to the figures. In the attached figures, the same or similar reference symbols are assigned to the same or similar components, and overlapping explanation of the same or similar components may be omitted in the explanation of respective embodiments. Further, a characteristic shown in each embodiment can be applied to the other embodiment as long as they are not contradictory to each other. Further, the figures are drawn in a schematic manner, so that actual sizes, ratios, and so on may not always coincide with those in the figures. Also, in the figures, a figure may include a part wherein relationship in terms of the size, the ratio, or the like is different from that relating to a corresponding part in the other figure.

### « 1. Examples of General Construction »

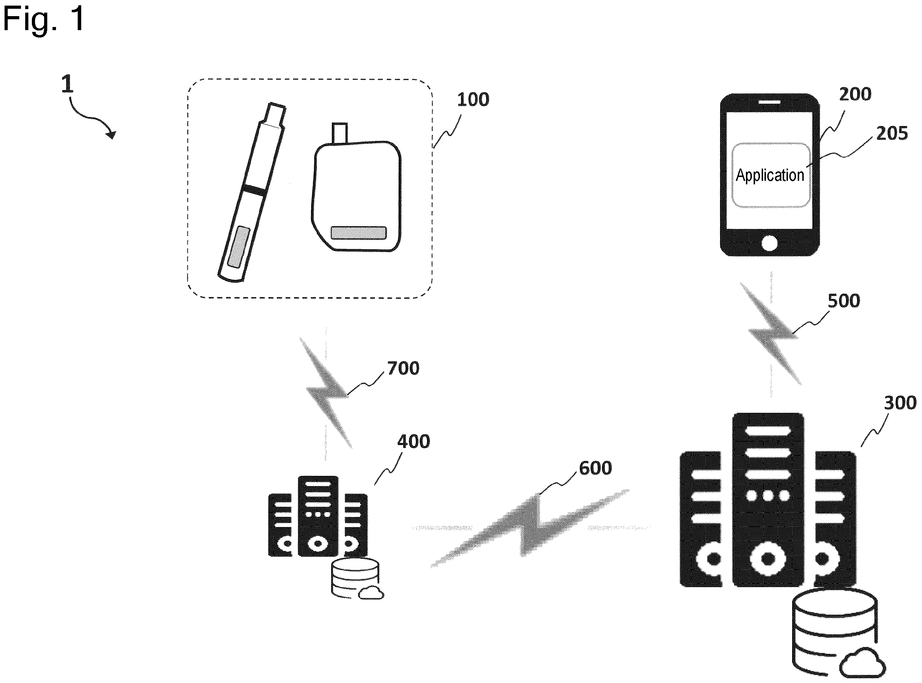

Fig. 1 is a figure which schematically shows a general construction of an information processing system according to an embodiment. An example system 1 comprises an aerosol generator 100, a user terminal 200, a server 300, an external server 400, and networks 500, 600, and 700.

The aerosol generator 100 is an example of an inhaler, and is an electronic device product which generates aerosol or flavor-added aerosol that is to be inhaled by a user. The aerosol generator 100 periodically transmits a communication request for communication with an external device.

The user terminal 200 is an example of an information processing terminal which obtains various kinds of information from the outside thereof and executes an application 205. The user terminal 200 transmits, to the aerosol generator 100, a setting request for setting operation of the aerosol generator 100.

The server 300 is an example of a server, and functions as a Web server and/or an application server for the user terminal 200 and the aerosol generator 100. That is, the server 300 provides a user with the application 205 via the Web. Further, the server 300 cooperates with the application 205 in the user terminal 200 and the aerosol generator 100 to perform management and so on of various kinds of data relating to a user and the aerosol generator 100. Still further, it performs management and so on of communication with the external server 400. In this regard, a part(s) of the function and the process of the application 205 may be performed by the server 300.

The external server 400 is an example of an external server, and intermediates between the aerosol generator 100 and the server 300 when data communication between them is performed. Specifically, the external server 400 transfers a communication request transmitted from the aerosol generator 100 to the server 300, and, in response to the communication request, transfers a setting request, that has been transmitted from the user terminal 200, from the server 300 to the aerosol generator 100.

In this regard, each of the server 300 and the external server 400 may be constructed by using a general-purpose computer such as a workstation, a personal computer, or the like. In the present case, each of the server 300 and the external server 400 may be constructed by using plural computers, and may be an aggregation of computers having various kinds of functions such as functions of a gateway, a database, a Web server, and so on. In this regard, a person killed in the art will understand that each of the server 300 and the external server 400 may be realized logically by using cloud computing.

The network 500 connects the user terminal 200 and the server 300 with each other by wireless communication, and an example thereof may be the Internet network.

The network 600 connects the server 300 and the external server 400 with each other by wireless communication, and an example thereof may be the Internet network.

The network 700 connects the external server 400 and the aerosol generator 100 with each other by wireless communication, and, in the present case, it may be a communication network conforming to an LPWA (Low Power Wide Area) wireless communication standard or a wireless communication standard defining communication regulations similar to those defined in the above standard (matters relating the above description will be explained later).

The system 1 constitutes a so-called IoT (Internet-of- Things) system. For example, in an IoT system, the aerosol generator 100 may constitute an IoT device, the external server 400 may constitute an IoT server (and an IoT gateway), and the network 700 may constitute an IoT network. Further, the server 300 may constitute an application server. Especially, in the case that the aerosol generator 100 performs transmission/reception of data by using a communication system that conforms to an LPWA wireless communication standard or a wireless communication standard defining communication regulations similar to those defined in the above standard, communication from the user terminal 200 to the aerosol generator 100 is performed in an asynchronous manner.

It should be reminded that, in addition to the mode shown in Fig. 1, the aerosol generator 100 may perform direct data communication with the user terminal 200 by using a short-range communication interface conforming to Bluetooth (a registered trademark), BLE (Bluetooth Low Energy), or the like. Further, data communication between the aerosol generator 100 and the user terminal 200 may be that realized by combining the above direct communication and the direct data communication through the Internet network via the above-explained various kinds of servers such as the servers 300 and 400.

In the following description, a hardware construction of each of the aerosol generator 100, the user terminal 200, the server 300, and the external server 400, which are components of the system 1, will be explained.

### < 1-1. Inhaler >

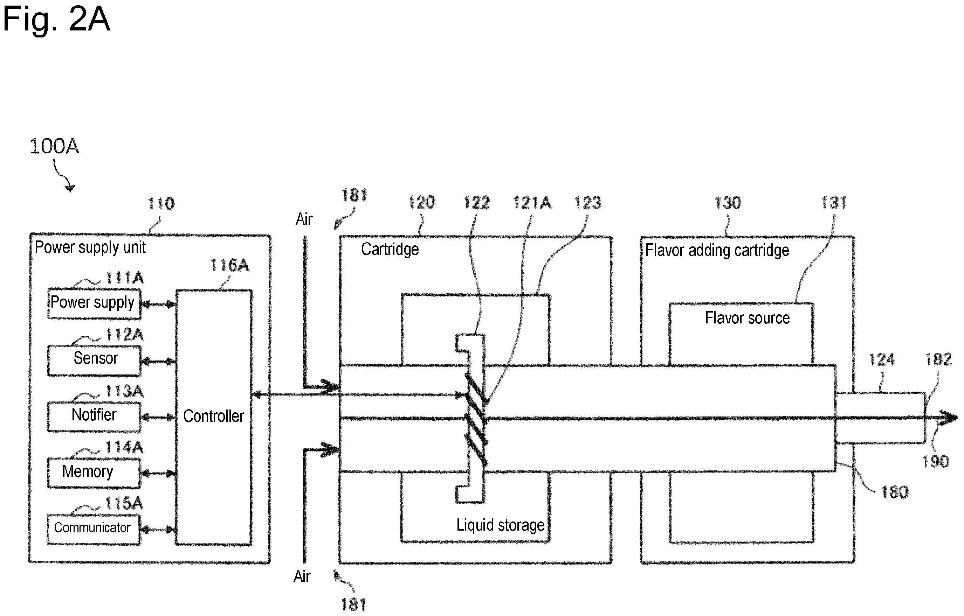

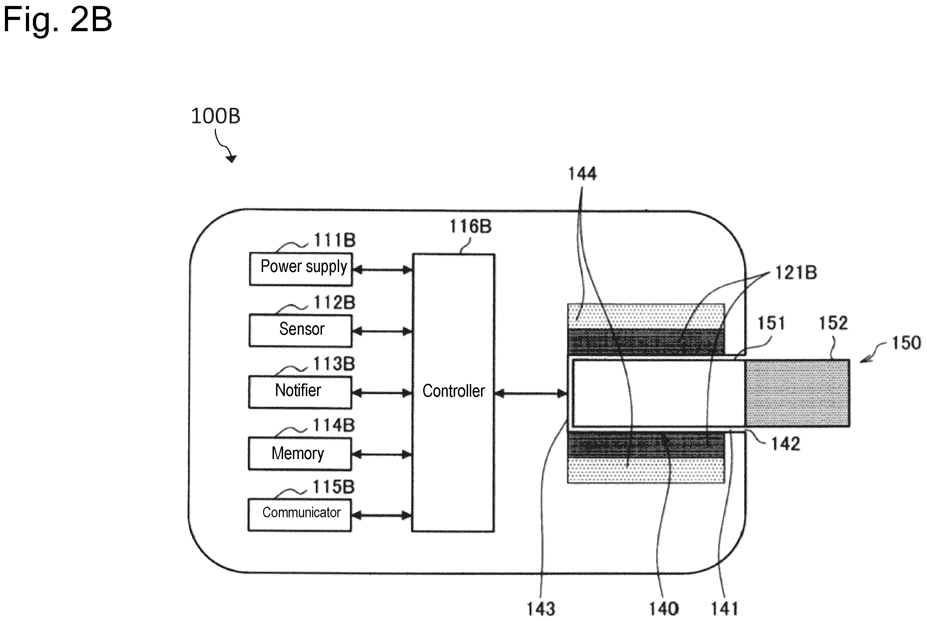

Each of Figs. 2A and 2B is a figure which schematically shows a construction example of the aerosol generator 100 which is an example of an inhaler. The aerosol generator 100 may be a heating-type flavor inhaler; and the inhalers may include various kinds of inhalers for generating aerosol or flavor-added aerosol inhaled by users. Further, generated inhaled components may include invisible vapor, in addition to aerosol.

### (1) First Construction Example

Fig. 2A is a schematic figure which schematically shows a first construction example of the aerosol generator 100. As shown in Fig. 2A, the aerosol generator 100A according to the present construction example comprises a power supply unit 110, a cartridge 120, and a flavor adding cartridge 130.

The power supply unit 110 comprises a power supply 111A, a sensor 112A, a notifier 113A, a memory 114A, a communicator 115A, and a controller 116A. The cartridge 120 comprises a heater 121A, a liquid guide 122, and a liquid storage 123. The flavor adding cartridge 130 comprises a flavor source 131 and a mouthpiece 124. An air flow path 180 is formed in each of the cartridge 120 and the flavor adding cartridge 130.

The power supply 111 stores electric power. The power supply 111 supplies, based on control by the controller 116A, electric power to respective components in the aerosol generator 100A. The power supply 111 may comprise, for example, a rechargeable battery such as a lithium-ion secondary battery or the like.

The sensor 112A obtains various kinds of information relating to the aerosol generator 100A. The sensor 112A may comprise a pressure sensor such as a microphone condenser or the like, a flow rate sensor, a temperature sensor, or the like, and obtains values relating to inhalation performed by a user. Further, the sensor 112A may comprise an input device such as a button, a switch, or the like which receives information input from a user.

The notifier 113A notifies a user of information. The notifier 113A may comprise, for example, a light emitting device which emits light, a display device for displaying images and/or texts, a sound outputting device for outputting sound, a vibration device which outputs vibration, or the like.

The memory 114A stores various kinds of information for operation of the aerosol generator 100A. The memory 114A comprises, for example, a nonvolatile storage medium such as a flash memory or the like. The memory 114A may comprise a volatile memory for providing a working area for control performed by the controller 116A.

The communicator 115A comprises, as explained above, a communication interface (including a communication module) that conforms to an LPWA wireless communication standard or a wireless communication standard defining communication regulations similar to those defined in the above standard. Further, the communicator 115A may comprise a communication interface which can perform communication conforming to any of other wired/wireless communication standards. For example, Wi-Fi (a registered trademark), Bluetooth (a registered trademark), or the like may be adopted as the communication standard.

The controller 116A functions as an arithmetic processing device and a control device, and controls overall operation of the aerosol generator 100A in accordance with various kinds of programs. For example, the controller 116A is realized by using an electronic circuit such as a CPU (Central Processing Unit), a microprocessor, or the like.

The liquid storage 123 stores an aerosol source. Aerosol is generated by atomizing the aerosol source. The aerosol source may be liquid such as polyhydric alcohol, such as glycerin, propylene glycol, or the like, or water, or the like, for example. The aerosol source may comprise a flavor component which is or is not originated from tobacco. In the case that the aerosol generator 100A is an inhaler for medical use such as a nebulizer or the like, the aerosol source may comprise a medicine.

The liquid guide 122 guides an aerosol source, which is a liquid stored in the liquid storage 123, from the liquid storage 123 and holds it. For example, the liquid guide 122 is a wick which is formed by twisting fiber material such as glass fibers or the like, or porous material such as porous ceramics or the like. In such a case, the aerosol source stored in the liquid storage 123 is guided by capillary effect occurring in the wick.

The heater 121A generates aerosol by atomizing an aerosol source by heating the aerosol source. In the example in Fig. 2A, the heater 121A comprises a coil that is wound around the liquid guide 122. As a result that heat is generated from the heater 121A, the aerosol source held in the liquid guide 122 is heated and atomized as a result thereof, and aerosol is generated accordingly. The heater 121A generates heat when electric power is supplied thereto from the power supply 111A. For example, supplying of electric power may be started, when one of or both starting of inhalation by a user and inputting of predetermined information is/are detected by the sensor 112A. Further, supplying of electric power may be stopped, when one of or both completion of inhalation by a user and inputting of predetermined information is/are detected by the sensor 112A.

The flavor source 131 is a component for adding flavor components to aerosol. The flavor source 131 may comprise a flavor component which is or is not originated from tobacco.

The air flow path 180 is a flow path for air inhaled by a user. The air flow path 180 has a tubular structure having two ends, i.e., an air inflow hole 181 which is an inlet for taking air into the air flow path 180, and air outflow hole 182 which is an outlet for releasing air from the air flow path 180. In the middle part of the air flow path 180, the liquid guide 122 is positioned on an upstream side (a side close to the air inflow hole 181), and the flavor source 131 is positioned on a downstream side (a side close to the air outflow hole 182). The air taken from the air inflow hole 181 during suction performed by a user is mixed with aerosol generated by the heater 121A, and conveyed to the air outflow hole 182 through the flavor source 131, as shown by an arrow 190. The flavor components included in the flavor source 131 are added to the aerosol, when a mixed fluid comprising the aerosol and the air passes through the flavor source 131.

The mouthpiece 124 is a member which is held in a user's mouth when suction action is performed. The air outflow hole 182 is positioned in the mouthpiece 124. A user can take the mixed fluid comprising the aerosol and the air into the user's mouth by holding the mouthpiece 124 in the user's mouth and performing suction action.

The construction example of the aerosol generator 100A has been explained in the above description. It is needles to state that the construction of the aerosol generator 100A is not limited to that explained above, and that the aerosol generator 100A may have any of various kinds of constructions shown below as examples.

For example, the aerosol generator 100A may not comprise the flavor adding cartridge 130. In such a case, the cartridge 120 is provided with the mouthpiece 124.

In a different example, the aerosol generator 100A may comprise plural kinds of aerosol sources. By mixing plural kinds of aerosol generated from the plural kinds of aerosol sources in the air flow path 180 to cause chemical reaction therein, a further different kind of aerosol may be generated.

Further, the means for atomizing an aerosol source is not limited to heating by the heater 121A. For example, the means for atomizing an aerosol source may be oscillation atomization or induction heating.

### (2) Second Construction Example

Fig. 2B is a schematic figure which schematically shows a second construction example of the aerosol generator 100. As shown in Fig. 2B, the aerosol generator 100B according to the present construction example comprises a power supply 111B, a sensor 112B, a notifier 113B, a memory 114B, a communicator 115B, a controller 116B, a heater 121B, a holding part 140, and a heat insulator 144.

Each of the power supply 111B, the sensor 112B, the notifier 113B, the memory 114B, the communicator 115B, and the controller 116B is substantially identical with each of the corresponding components included in the aerosol generator 100A according to the first construction example. Especially, the communicator 115B comprises, as explained above, a communication interface (including a communication module) that conforms to an LPWA wireless communication standard or a wireless communication standard defining communication regulations similar to those defined in the above standard.

The holding part 140 comprises an inner space 141, and holds a stick-type base material 150 by housing a part of the stick-type base material 150 in the inner space 141. In this regard, the stick-type base material 150 is also an example of a "refill." The holding part 140 comprises an opening 142 which makes the inner space 141 communicate with the outside, and holds the stick-type base material 150 inserted from the opening 142 into the inner space 141. For example, the holding part 140 has a cylindrical shape having the opening 142 and a bottom part 143 which is a bottom plane, and defines a columnar inner space 141. The holding part 140 also has a function to define a flow path of air supplied to the stick-type base material 150. An air inflow hole, which is an inlet of air to the above flow path, is formed in the bottom part 143, for example. On the other hand, an air outflow hole, which is an outlet of air from the above flow path, is the opening 142.

The stick-type base material 150 comprises a base material part 151 and a suction opening part 152. The base material part 151 comprises an aerosol source. In the present construction example, the form of the aerosol source is not limited to liquid, and it may be solid. In the state that the stick-type base material 150 is being held by the holding part 140, at least a part of the base material part 151 is housed in the inner space 141, and at least a part of the suction opening part 152 protrudes from the opening 142. Thus, when the suction opening part 152, which protrudes from the opening 142, is held in a user's mouth and suction action is performed by the user, air flows into the inner space 141 from the air inflow hole which is not shown in the figure, and arrives at the inside of the user's mouth, together with aerosol generated from the base material part 151.

The heater 121B comprises a construction similar to that of the heater 121A according to the first construction example. However, in the example shown in Fig. 2B, the heater 121B is constructed to have a film shape, and is arranged to cover the outer periphery of the holding part 140. Thus, when heat is generated by the heater 121B, the base material part 151 of the stick-type base material 150 is heated from the outer periphery thereof, and aerosol is generated accordingly.

The heat insulator 144 prevents heat transfer from the heater 121B to other components. For example, the heat insulator 144 comprises vacuum insulation material, aerogel insulation material, or the like.

In the above description, a construction example of the aerosol generator 100B has been explained. Although it is needless to state, the construction of the aerosol generator 100B is not limited to that explained above, and it may have any of various constructions shown below as examples.

For example, the heater 121B may be constructed to have a blade shape, and arranged to protrude from the bottom part 143 to the inner space 141 of the holding part 140. In such a case, the blade-shape heater 121B is inserted into the base material part 151 of the stick-type base material 150, and the base material part 151 of the stick-type base material 150 is heated from the inside thereof. In a different example, the heater 121B may be arranged to cover the bottom part 143 of the holding part 140. Further, the heater 121B may be constructed as that comprising a combination of two or more of a first heater covering the outer periphery of the holding part 140, a second heater having a blade shape, and a third heater covering the bottom part 143 of the holding part 140.

In a different example, the holding part 140 may comprise an opening/closing mechanism, such as a hinge or the like, for opening/closing a part of an outer shell which forms the inner space 141. The holding part 140 may hold the stick-type base material 150 inserted into the inner space 141, by opening/closing the outer shell. In such a case, the heater 121B may be arranged in the position where the stick-type base material 150 is to be held in the holding part 140, and may heat the stick-type base material 150 while it is being pressed thereby.

Further, the means for atomizing the aerosol source is not limited to heating by the heater 121B. For example, the means for atomizing the aerosol source may be induction heating.

Further, the aerosol generator 100B may comprise the heater 121A, the liquid guide 122, the liquid storage 123, and the air flow path 180 according to the first construction example, and the air outflow hole 182 of the air flow path 180 may double as an air inflow hole to the inner space 141. In such a case, the fluid mixture comprising air and aerosol generated by the heater 121A flows into the inner space 141, is further mixed with aerosol generated by the heater 121B, and arrives at the inside of a user's mouth.

### < 1-2. Information Processing Terminal >

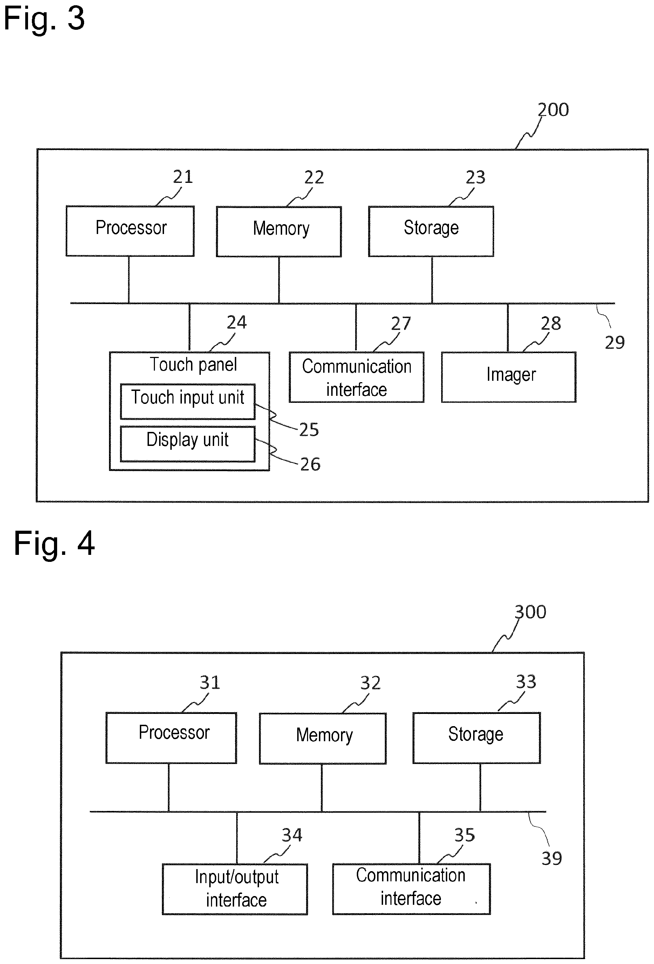

Fig. 3 is a figure which schematically shows a hardware construction example of the user terminal 200 which is an example of an information processing terminal. The user terminal 200 may be an electronic device product such as a smartphone, a tablet terminal, a laptop computer, a personal computer, or the like, for example. In this regard, it is supposed that the example of the user terminal 200 shown in Fig. 3 is a smartphone.

The user terminal 200 comprises a processor 21, a memory 22, a storage 23, a touch panel 24, a communication interface 27, and an imager 28. These components are electrically connected with one another via a bus 29.

The processor 21 is an arithmetic processing device which controls information processing for controlling transmission/reception of data between respective components, and preforming an information processing method according to an embodiment. For example, the processor 21 is a CPU, and executes a program and so on, such as the application 205 stored in the storage 23 and unfolded in the memory 22, to perform the information processing method according to the embodiment. Further, the processor 21 in the user terminal 200 may comprise, in addition to the CPU, a GPU (Graphical Processing Unit) and so on.

The memory 22 comprises a main memory which is realized by using a volatile memory device such as a DRAM (Dynamic Random Access Memory) or the like, and an auxiliary storage realized by using a nonvolatile memory device such as a flash memory, a HDD (Hard Disc Drive), or the like. The memory 22 is used as a work area of the processor 21 or the like, and stores a BIOS (Basic Input/Output System) executed at the time of activation of an OS (operating system), various kinds of setting information, and so on.

The storage 23 stores programs, an OS, and so on for information processing performed by the processor 21. Further, a database, which stores various kinds of data used when executing programs, may be constructed in the storage 23.

The touch panel 24 is a component which constitutes an input/output interface. The touch panel 24 comprises a touch input unit 25 and a display unit 26. The touch input unit 25 receives touch manipulation that is performed by a user and applied to the touch panel 24. The display unit 26 is realized by using a liquid crystal display or the like.

The communication interface 27 connects the user terminal 200 to the network 500, and allows communication with external devices such as the server 300 and so on. The communication interface 27 comprises a communication module which conforms to a communication standard of the third generation mobile communication system (3G), the fourth generation mobile communication system (4G), the fifth generation mobile communication system (5G), LTE (Long Term Evolution), a wireless LAN, or the like, for example. The communication module connects, in a wireless manner, to a network such as a Wi-Fi or the Internet network. Further, the communication interface 27 may comprise a short-range communication interface conforming to Bluetooth (a registered trademark), BLE (Bluetooth Low Energy), or the like, and directly communicate with an external device.

The imager 28 comprises a front camera and a rear camera in the user terminal 200. That is, the imager 28 has a camera function, and stores images and movies taken by a user in the storage 53 or the like.

The bus 29 connects respective components with one another, and transmits address signals, data signals, and control signals. It will be understood by a person skilled in the art that the user terminal 200 may comprise, in addition to the above components, various kinds of components. For example, it may comprise a position information obtaining means for obtaining information of the position of the user terminal 200, and a quantity-of-activity obtaining means for obtaining quantities of activity performed by a user (for example, the number of steps, the distance travelled, and so on).

### < 1-3. Server >

Fig. 4 is a figure which schematically shows a hardware construction example of the server 300 which is an example of a server. The server 300 comprises a processor 31, a memory 32, a storage 33, an input/output interface 34, and a communication interface 35, and these components are electrically connected with one another via a bus 39.

The processor 31, the memory 32, the storage 33, the communication interface 35, and the bus 39 are substantially similar to the processor 21, the memory 22, the storage 23, the communication interface 27, and the bus 29 in the user terminal 200 shown in Fig. 3, respectively. In the server 300, the input/output interface 34 receives input from information inputting devices such as a mouse, a keyboard, and so on, and also supplies output to outputting devices such as a display and so on.

Further, a hardware construction of the external server 400, which is an example of an external server, is substantially similar to that of the server 300.

### < 1-4. Data Communication between Inhaler and External Server >

As explained above, the network 700 between the aerosol generator 100 and the external server 400 is a communication network which conforms to an LPWA wireless communication standard or a wireless communication standard defining communication regulations similar to those defined in the above standard (hereinafter, this may be simply referred to as "LPWA wireless communication standard"). In this regard, Sigfox, LoRA-WAN, or the like may be adopted as the LPWA wireless communication standard. That is, the aerosol generator 100 comprises a communication module conforming to the LPWA wireless communication standard, and data communication between the external server 400 and the aerosol generator 100 is performed according to a communication protocol conforming to the LPWA wireless communication standard.

In the following description, data communication between the aerosol generator 100 and the external server 400 will be explained. In this regard, in the following explanation, Sigfox is adopted as an example of the LPWA wireless communication standard. The network 700, in which Sigfox has been adopted, is used as a private network which uses radio signals in a 920 MHz band. The aerosol generator 100 comprises a Sigfox communication module for accessing the network 700. Further, the external server 400 constitutes a Sigfox server/cloud (which is operated by the Sigfox S.A.). It should be reminded that the LPWA wireless communication standard is not limited to Sigfox, and a person skilled in the art will understand that any wireless communication standard defining regulations similar to those defined in Sigfox can be adopted for communication.

The data communication between the aerosol generator 100 and the external server 400 is one-to-one communication between the aerosol generator 100 and the external server 400, and is based on the communication protocol defined in Sigfox. It should be reminded that the communication module included in the aerosol generator 100 is associated with a device ID, wherein a device ID is that used for uniquely identifying a device.

First, the aerosol generator 100 generates, by using the device ID thereof, a communication request directed to the external server 400. The communication request is periodically transmitted from the aerosol generator 100 to the externals server 400, to make the external server 400 allow data communication (that is in a backward direction) from the external server 400 to the aerosol generator 100. That is, by using an event of receiving the communication request from the aerosol generator 100 as a trigger, the external server 400 allows itself to perform data communication to the aerosol generator 100. That is, the externals server 400 does not start data communication to the aerosol generator 100, initiatively.

After receiving the communication request from the aerosol generator 100, the external server 400 records the device ID of the aerosol generator 100, and allows data communication to the aerosol generator 100 for a predetermined allowed period of time. During the predetermined allowed period of time, the external server 400 allows data, that is directed from the server 300 to the aerosol generator 100, to pass through the external server 400 only one time.

As explained above, some restrictions on data communication between the aerosol generator 100 and the external server 400 are defined. The restrictions herein include the following matters, for example.
(a) With respect to transmission of a communication request generated by the aerosol generator 100, the transmission is allowed to be performed only a predetermined number of times within a predetermined period of time.
(b) With respect to data communication from the external server 400 to the aerosol generator 100, the data communication is allowed to be performed only within a predetermined allowed period of time that starts at each predetermined time interval.
(c) With respect to data communication from the external server 400 to the aerosol generator 100, the data communication is allowed to be performed only a predetermined number of times within a predetermined period of time .

In more detail, regarding above item (a), i.e., regarding data communication from the aerosol generator 100 to the external server 400, it is defined with respect to time and the number of times of data communication that the maximum number of times of data communication per day is 140. That is, in the case that the aerosol generator 100 performs action for requesting communication 140 times periodically per day, it can be understood that the action for requesting communication is performed one time per approximately 10 minutes. In this regard, it is not necessarily required to perform periodical transmission of the communication request.

Further, regarding item (b), i.e., regarding data communication from the external server 400 to the aerosol generator 100, it is restricted in such a manner that the data communication is allowed to be performed only within a predetermined allowed period of time that starts after acceptance of a communication request from the aerosol generator 100. For example, data communication from the external server 400 to the aerosol generator 100 is restricted in such a manner that data communication is allowed to be performed within 10 seconds after a communication request from the aerosol generator 100 is accepted. That is, in the case that a communication request is received from the aerosol generator 100 at a frequency similar to that relating to above item (a), i.e., one time per approximately 10 minutes, it can be understood that data communication to the aerosol generator 100 is allowed to be performed only within 10 seconds per every 10 minutes. In this regard, if the allowed period of time has elapsed before commencing of data communication, a time-out occurs, and, in general, data communication from the external server 400 to the aerosol generator 100 is prohibited until the time of a next allowed period of time.

Further, regarding item (c), i.e., regarding data communication from the external server 400 to the aerosol generator 100, it is defined with respect to time and the number of times of data communication that the maximum number of times of data communication per day is 4. That is, the external server 400 is allowed to perform at most 4 times of data communication to the aerosol generator 100 per day. In the case that data communication has been performed 5 times or more in a day, the fifth data communication and data communication following thereto are discarded or carried over to a next day.

Also, a person skilled in the art will understand that there is a restriction with respect to the quantity of data relating to a single data transmission/reception process, in addition to the above restrictions.

### < 1-5. Data Communication between External Server and Server >

Regarding data communication between the external server 400 and the serve 300, HTTP (Hyper Text Transfer Protocol) communication via the network 600 (for example the Internet network) is adopted therein. As explained above, since restrictions relating to data communication between the aerosol generator 100 and the external server 400 have been defined in the LPWA wireless communication standard, it is required that the function of the server 300 be designed to make it work as an application server for corresponding to the above restrictions.

Specifically, after receiving a communication request from the aerosol generator 100, the external server 400 transfers the communication request to the server 300. On the other hand, the server 300 performs action to respond to the communication request from the external server 400 in such a manner that the action matches the allowed period of time in the external server 400. In more detail, the server 300 holds transmission of a setting request, that has been received from the user terminal 200, to the external server 400, and, according to the allowed period of time, transmits the setting request to the external server 400 as a response to the communication request from the external server 400.

### « 2. Information Processing System »

In the following description, the process performed in the information processing system according to the present embodiment will be explained in detail. As explained above, in the information processing system according to the present embodiment, the aerosol generator 100 comprises a communication module conforming to the LPWA wireless communication standard, and communication between the external server 400 and the aerosol generator 100 is performed according to a communication protocol conforming to the LPWA wireless communication standard. Predetermined restrictions have been defined with respect to communication between the external server 400 and the aerosol generator 100, and the end-to-end communication from the user terminal 200 to the aerosol generator 100 is performed in an asynchronous manner.

Thus, in the case that a user performs operation setting of the aerosol generator 100 remotely by using the user terminal 200, some length of time may be required until a setting request arrives at the aerosol generator 100. Accordingly, there is a case wherein a time lag having some time length may exists between the time when a user has completed operation setting of the aerosol generator 100 on the user terminal 200 and the time when the setting is actually reflected to the aerosol generator 100.

A user performs operation setting of the aerosol generator 100 via the application 205 in the user terminal 200. On the other hand, in the case that occurrence of a time lag relating to operation setting is taken into consideration, it is preferable to provide usability that prevents a user from applying unintended operation setting to the aerosol generator 100, so as to prevent erroneous input manipulation performed by the user.

The information processing system according to the present embodiment provides a user with a user interface which presents, to the user, the newest status with respect to whether operation setting of the aerosol generator 100 has been actually reflected in the aerosol generator 100, and, in the case that the operation setting has not yet been reflected, prevents reception of further input for operation setting. By the above construction, it becomes possible to prevent a user from performing erroneous manipulation, and prevent a user from applying unintended setting to the aerosol generator 100.

### < 2-1. Functional Construction Example >

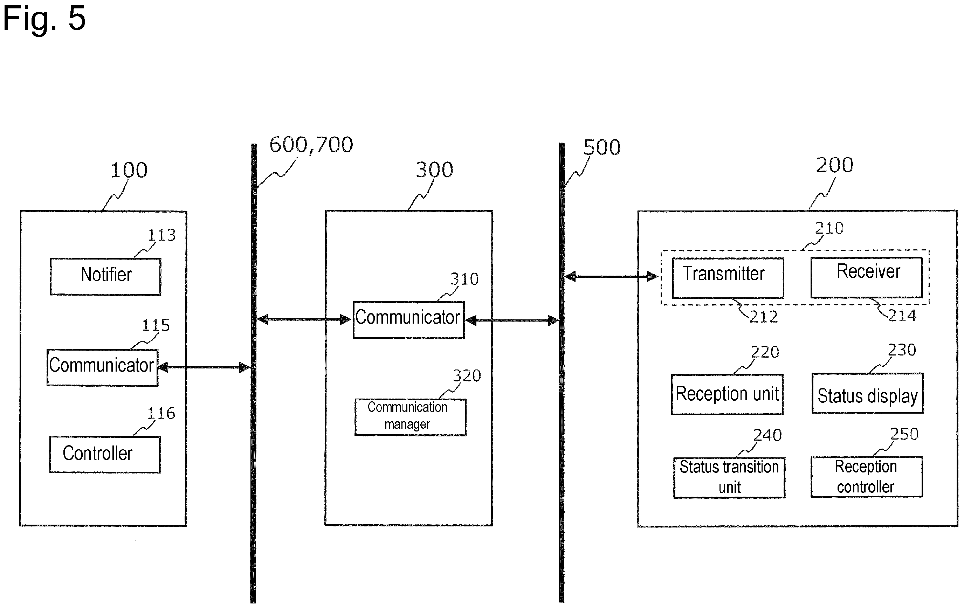

Fig. 5 is a figure which schematically shows, in the forms of blocks, functional constructions implemented in an information processing system according to a present embodiment. Specifically, an information processing system 1, which is used when performing operation setting of the aerosol generator 100 remotely from the user terminal 200, comprises the aerosol generator 100 shown in Fig. 2A or 2B, the user terminal 200 shown in Fig. 3, and the server 300 shown in Fig. 4.

Fig. 5 shows, in the forms of blocks, main functional constructions implemented in respective components in the aerosol generator 100, the user terminal 200, and the server 300. For simplicity, depicting of the external server 400 shown in Fig. 1 is omitted herein. In this regard, a person skilled in the art will understand that the functional constructions are not limited to those explained above, and addition, deletion, modification, or the like of functional constructions is possible optionally.

The aerosol generator 100 comprises a notifier 113, a communicator 115, and a controller 116 as shown in each of Figs. 2A and 2B.

The notifier 113 comprises a display device for displaying images and/or texts. For example, information inputted by a user by using the user terminal 200 may be received, and the received information may be displayed by the notifier 113.

The communicator 115 transmits a communication request periodically to the external server 400, according to restrictions in the above-explained LPWA wireless communication standard. Further, the communicator 115 receives, from the server 300 via the external server 400, a setting request relating to operation setting of the aerosol generator 100. The setting request is constructed as a response to the communication request. Further, the communicator 115 transmits, to the external server 400, a completion confirmation that represents the state that the aerosol generator 100 has completed operation setting.

The controller 116 performs operation setting in the aerosol generator 100, in response to reception of the setting request by the communicator 115. That is, operation setting in the aerosol generator 100 is actually performed for responding to the setting request from the user terminal 200.

The user terminal 200 comprises a communicator 210, a reception unit 220, a status display 230, a status transition unit 240, and a reception controller 250.

The communicator 210 comprises the communication interface 27 shown in Fig. 3, and comprises a transmitter 212 and a receiver 214. The transmitter 212 in the communicator 210 transmits, to the server 300, a setting request that is directed to the aerosol generator 100. The receiver 214 receives, from the aerosol generator 100 via the server 300, a completion confirmation that represents the state that the aerosol generator 100 has completed operation setting. Further, regarding the restriction of data communication relating to the aerosol generator 100 in the LPWA wireless communication standard, the receiver 214 receives, from the server 300, data restriction information including the length of waiting time until the time when the above restriction is removed.

The reception unit 220 comprises the touch panel 24 (especially, the touch input unit 25) shown in Fig. 3, the imager 28, a sound inputting means (which is not shown in the figures), and so on. The reception unit 220 accepts, via input manipulation performed by a user, a setting request to the aerosol generator 100.

The status display 230 comprises the touch panel 24 (especially, the touch input unit 25) shown in Fig. 3. The status display 230 displays, on the application 205 in the user terminal 200, an operation status of the aerosol generator 100 put in a remote position.

The status transition unit 240 comprises the processor 21 shown in Fig. 3. The status transition unit 240 changes, in response to transmission of a request by the transmitter 212, a display status relating to the aerosol generator 100 to the status "Waiting for Reflection" and makes the status display 230 display the changed display status. Further, it changes, in response to a completion confirmation from the aerosol generator 100, the display status from the status "Waiting for Reflection" to the status "Reflection Completed" and makes the status display 230 display the changed display status.

The reception controller 250 comprises the processor 21 shown in Fig. 3. During a period of time when the display status shown by the status display 230 is the status "Waiting for Reflection," the reception controller 250 prohibits accepting of a further setting request relating to operation setting of the aerosol generator 100. Further, it allows accepting of a further setting request, in response to changing of the display status shown by the status display 230 from the status "Waiting for Reflection" to the status "Reflection Completed."

The server 300 comprises a communicator 310 and a communication manager 320.

The communicator 311 comprises the communication interface 35 shown in Fig. 4. The communicator 310 transmits a setting request, that has been received from the user terminal 200, to the aerosol generator 100 via the external server 400. Further, the communicator 310 transmits a completion confirmation, that has been received from the aerosol generator 100 via the external server 400, to the user terminal 200. Further, the communicator 310 transmits a restriction information to the user device 200.

The communication manager 320 comprises the processor 31 shown in Fig. 4. The communication manager 320 manages, based on predetermined restrictions in the LPWA wireless communication standard, transmission of a setting request performed by the communicator 310. Specifically, in response to reception of a setting request by the communicator 310, it judges whether communication between the external server 400 and the aerosol generator 100 is restricted, and, if it is judged that communication has been restricted, makes the communicator 310 hold transmission of the setting request to the aerosol generator 100. Further, the communication manager 320 determines a restriction information that is based on the above restriction and relates to the setting request. The restriction information includes the length of waiting time until the time when the restriction of communication is removed. That is, the communication manager 320 calculates the waiting time, based on information of the restriction relating to the LPWA wireless communication standard.

### < 2-2. Example of Process Flow >

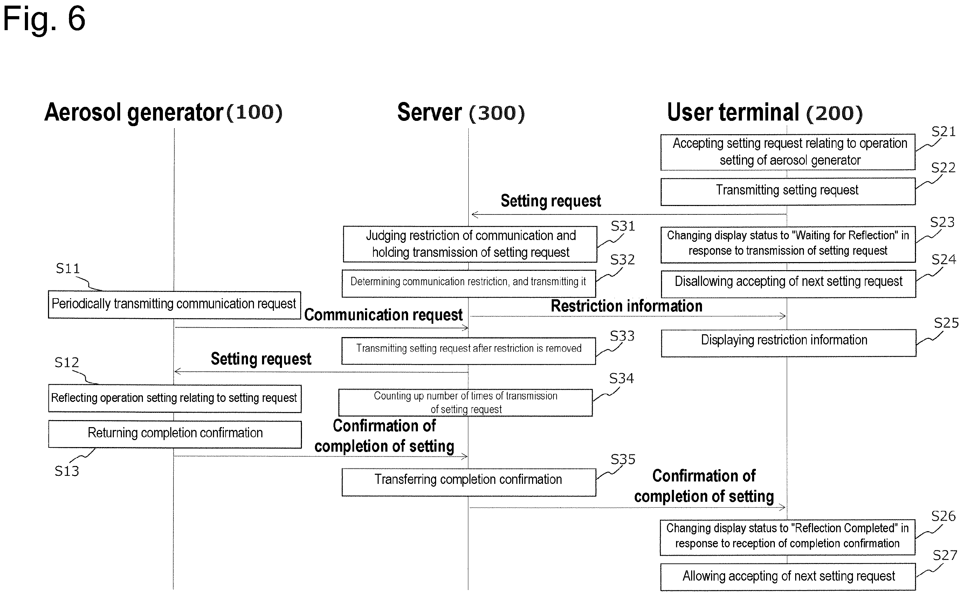

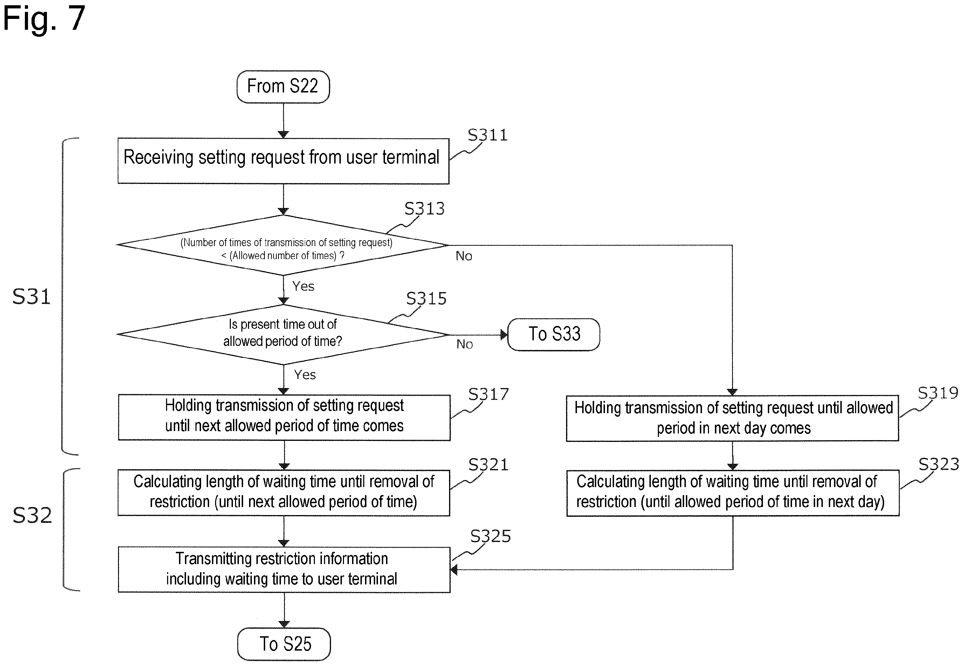

An example of a process flow in an information processing system according to a present embodiment will be explained with reference to Figs. 6 and 7. Specifically, the following description shows examples of processing steps that make it possible to perform operation setting of the aerosol generator 100 remotely by a user by using the user terminal 200. Fig. 6 is a figure of an example of a processing flow in the information processing system according to the present embodiment. Fig. 7 is a figure of an example of a detailed flow of processing steps included in some parts shown in Fig. 6.

It should be reminded that the respective steps shown in the flow charts relating to the present specification are mere examples, and the steps are not limited to the above respective steps, so that one or plural different processing steps may be included therein and/or one or plural processing steps may be omitted therefrom, optionally. Further, a person skilled in the art will understand that the order of the respective steps is a mere example order, and the order is not limited to the above order, so that there may be cases wherein the respective steps can be arranged in an arbitrarily selected order, and/or some of the respective steps can be performed in a parallel manner.

First, in the sequence of processing steps, the reception unit 220 in the user terminal 200 accepts a setting request relating to operation setting of the aerosol generator 100 (step S21).

In response to acceptance of the setting request in step S21, the transmitter 212 in the user terminal 200 transmits, to the server 300, the setting request directed to the aerosol generator 100 (step S22).

Next, in response to transmission of the setting request in step S22, the status transition unit 240 in the user terminal 200 changes the display status relating to the aerosol generator 100 to the status "Waiting for Reflection" (step S23).

Thereafter, during the period of time when the display status is "Waiting for Reflection," the reception controller 250 disallows accepting of a further setting request relating to operation setting of the aerosol generator 100 (step S24).

On the other hand, in the server 300 which has received the setting request, the communication manager 320 judges, based on predetermined restrictions in the LPWA wireless communication standard, a restriction, that is to be applied, of communication, and holds transmission of the setting request (step S31).

Next, the communication manager 310 in the server 300 determines, according to the applied communication restriction, restriction information, and transmits the restriction information to the user terminal 200 (step S32).

Steps S31 and S32 will be explained in more detail with reference to Fig. 7. As shown in Fig. 7, regarding the setting request transmitted from the user terminal 200 in step S22, the communicator 310 in the server 300 receives the setting request (step S311).

Next, the communication manager 320 performs judgment relating to applying of a communication restriction to the received setting request. Specifically, first, it performs judgment as to whether the number of times of transmission to the aerosol generator 100 at present is less than an allowed number of times (step S313). If the number of times of transmission at present is less than the allowed number of times (Yes), the communication manager 320 further performs judgment as to whether the present time is that out of an allowed period of time (step S315). The allowed number of times and the allowed period of time used herein have been related to the restriction of data communication of the aerosol generator 100 in the LPWA wireless communication standard.

As explained above, in an example, the allowed number of times of data communication relating to the aerosol generator 100 is at most 4 times per day. That is, in step S313, by performing judgment as to whether the number of times of transmission at present is less than 4 times, it is judged whether a received setting request is allowed to be transmitted within the same day.

Further, in an example, the period of time during that the data communication relating to the aerosol generator 100 is allowed is limited to 10 seconds given in relation to a communication request that is accepted every 10 minutes from the aerosol generator 100. That is, in step S315, by performing judgment as to whether the present time is that out of the allowed period of time that is given every 10 minutes and has the length of 10 seconds, it is judged whether responding to the communication request is allowed at the present time.

In the case that result of each of steps S313 and S315 is "Yes," the communication manager 320 holds transmission of the setting request until a next allowed period of time comes (step S317).

On the other hand, in the case that result of step S313 is "No," it is interpreted that the number of times exceeds the number of times allowed within a day when the setting request was received, so that the communication manager 320 holds transmission of the setting request for a period of time until a next day of the above day comes (step S319).

In this regard, if it is judged in step S315 that the present time is that within the allowed period of time (No), the communication manager 320 can allow transmission of the setting request by the communicator 310 without further qualification (step S33 that will be explained later).

Next, the communication manager 320 calculates, based on the present time, the allowed number of times, and the allowed period of time, the length of waiting time until the time when the restriction relating to holding of transmission of the setting request is removed.

Specifically, regarding step S317, the communication manager 320 calculates the length of waiting time until the time when a next allowed period of time comes (step S321). It is understood that the waiting time herein is less than 10 minutes.

On the other hand, regarding step S319, the communication manager 320 calculates the length of waiting time until the time when a first allowed period of time in a next day comes (step S323). It is understood that the waiting time herein is less than 24 hours.

The waiting time calculated in each of steps S321 and S323 is transmitted, for presenting it to a user, to the user terminal 200 by the communicator 310 as a part of control information (S325). In this regard, in the user terminal 200, it is preferable to display the waiting time in real time in a counting down manner. Further, in addition to the waiting time, it may be possible to send, as a part of the control information and to the user terminal, a remaining number of times that the setting request can be made.

Regarding the explanation of Fig. 7, there may be plural number of setting requests which are waiting for transmission thereof. Specifically, after reception of a setting request from a user terminal 200 in step S311, the communication manager 320 registers the setting request with a queue, and can relate respective users to respective queues and manage them. In such a case, the setting requests, that are in waiting states, are transmitted sequentially when their turns to be processed come, respectively. In a different construction, in the case that there are plural number of setting requests which are waiting for transmission thereof, the communication manager 320 may select a first-received setting request as the only object of transmission, and discard setting requests received after receiving of the first-received setting request.

Next, Fig. 6 will be referred to again. After transmission of the restriction information from the server 300 to the user terminal 200 in step S32, the status display 230 in the user terminal 200 displays the restriction information (especially, the information relating to waiting) on the application 205. By the above construction, a user can grasp, after transmission of the setting request to the server 300 in step S22, the length of waiting time until the time of removing of the restriction. That is, the user can roughly grasp the length of time that is required until the time when reflecting of the setting in the aerosol generator 100 is completed.

It should be reminded that, through the processes in steps S21-S25 in the user terminal 200 and the processes in steps S31 and S32 in the server 300, the communicator 115 in the aerosol generator 100 continues action to periodically transmit the communication request (step S11). In an example, as explained above, the communicator 115 transmits the communication request 140 times per day, i.e., one time at a time interval of approximately 10 minutes. The communication request is received by the server 300 via the external server 400.

The communication manager 320 in the server 300 confirms a communication request from the aerosol generator 100. If it is determined as a result thereof that there is a setting request that should be returned to the aerosol generator 100 as a response to the communication request, the communicator 310 transmits the setting request to the aerosol generator 100 at timing when the restriction is removed (step S33).

After transmitting of the setting request by the communicator 310, the communication manager 320 counts up (+1) the number of times of transmission of the setting request (step S34). It is preferable to use the number of times counted herein in the process in step S313 for performing judging relating to effectiveness of the restriction.

The setting request transmitted in step S33 is received by the aerosol generator 100 via the external server 400. For responding to the setting request, the controller 116 in the aerosol generator 100 actually reflects therein the operation setting relating to the setting request (step S12).

After reflecting of the operation setting in step S12, the communicator 115 in the aerosol generator 100 returns, to the server 300, a completion confirmation that represents the state that the aerosol generator 100 has completed the operation setting (step S13).

Thereafter, the communicator 310 in the server 300, which has received the completion confirmation, transfers the completion confirmation to the user terminal 200 (step S35).

In response to reception of the completion confirmation by the receiver 214 in the user terminal 200, the status transition unit 240 changes the display status from "Waiting for Reflection" to "Reflection Completed" (step S26). As a result, a user can confirm, on the application 205, completion of reflection of the registered operation setting in the aerosol generator 100.

Finally, regarding accepting of a next setting request that has been set to be disallowed in step S24, the reception controller 250 in the user terminal 200 may change the setting to allow accepting of the next setting request (step S27). By controlling operation for accepting a setting request by the reception controller 250 as explained above, it becomes possible to prevent a user from performing erroneous manipulation of the user terminal 200 and accordingly prevent the aerosol generator 100 from performing erroneous operation setting.

In this regard, the reception controller 250 may also use the information relating to waiting, that has been received in step S25, to control whether operation for receiving a next setting request will be performed. For example, in the case that the number of times has already exceeded the allowed number of times, the reception controller 250 does not switch, in step S27, its state immediately to a state wherein accepting of a next setting request is allowed, and may maintain, until a next day, the state wherein accepting of a next setting request is disallowed.

### < 2-3. Examples of Screen Images >

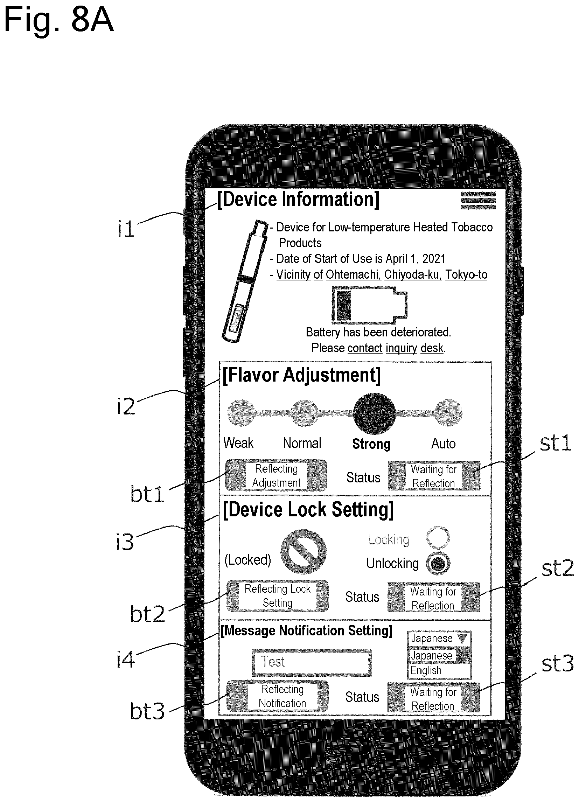

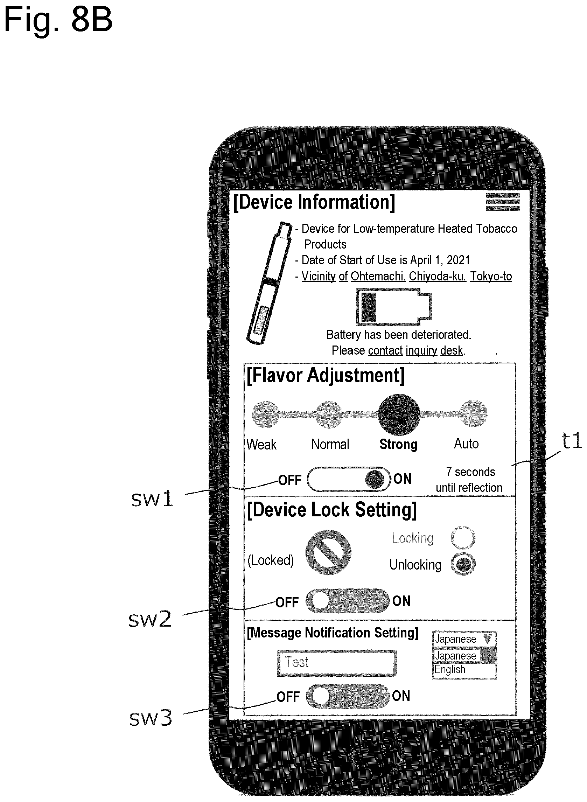

Figs. 8A and 8B show examples of screen images of the application 205 displayed by the user terminal 200. Regarding the screen of the application 205 on the user terminal 200, it is displayed on the touch panel 24, and input manipulation performed by a user is accepted thereby.

As shown in the example in Fig. 8A, the display on the screen comprises device information display i1, flavor adjustment display i2, device lock setting display i3, and message notification setting display i4.

The device information display i1 comprises various kinds of information of the aerosol generator 100 that has been registered with the server 300. In the present case, "Device for Low-temperature Heated Tobacco Products" is displayed as the kind of the device, and the date "April 1, 2021 " is displayed as the data of a start of use of the device, wherein the data is that when registration of the device is performed and the device is used for the first time by the user. The above information is obtained, via the application 205, at the time when registration of the aerosol generator 100 is performed by a user, and stored in the database in the server 300.

Further, by providing the network 700, which conforms to the LPWA wireless communication standard, with a radio base station (which is not shown in the figures), it becomes possible to locate the position of the aerosol generator 100 comprising a communication module corresponding to the LPWA wireless communication standard. The specified position information is transmitted to the server 300 via the external server 400. The device information display i1 may comprise the position of the aerosol generator 100 such as that explained above, and, in the exampled in Fig. 8A, the display "Vicinity of Ohtemachi, Chiyoda-ku, Tokyo" is included therein. In this regard, the position information may be associated with a map application to display the position information in the form of a map.

Further, the device information display i1 may comprise the remaining power of a battery in the aerosol generator 100. Regarding information of the remaining power of the battery in the aerosol generator 100, a user terminal 200 may send a request for acquisition of the information to the aerosol generator 100 at the time of activation of the application 205, and, in response thereto, the information may be obtained from the aerosol generator 100 via the external server 400. In a different construction, the controller 116 in the aerosol generator 100 may successively monitor the remaining power of the battery, and, by using, as a trigger for transmission, detection of a state that the value of the remaining power of the battery has become that below a predetermined threshold value, may transmit information to the user terminal 200 via the external server 400. In the example in Fig. 8A, the message "Battery has been deteriorated. Please contact the inquiry desk." is displayed together with an indicator of the remaining power of the battery.

In the flavor adjustment display i2, a user can set, by the user, a profile of heating operation in the aerosol generator 100. In the present case, a user can select, from profiles "Weak," "Normal," "Strong," and "Auto," a profile that the user prefers, and set it in the aerosol generator 100. The selected profile is validated by setting it as setting information in the memory 114 in the aerosol generator 100. In the example in Fig. 8A, a user is attempting to set the profile of the aerosol generator 100 to "Strong."

In the device lock setting display i3, a user can set operation of the aerosol generator 100 with respect to locking or unlocking. In this regard, for example, during a period of time when the aerosol generator 100 is in a locked state, the power supply of the aerosol generator 100 is not turned on, and, thus, the aerosol generator 100 does not perform operation thereof, even if a power source button is pressed. That is, if an unlocking action is not performed by a user by using the user terminal 200, the aerosol generator 100 will not operate actually. Regarding the example in Fig. 8A, it is displayed therein, together with an icon, that the state of the aerosol generator 100 at present is "Locked." Further, a user is attempting to change the state of the aerosol generator 100 to "Unlocked." In this regard, since the state of the aerosol generator 100 at present is "Locked," the display of the "Locking" button is deactivated to make it unselectable.

In the message notification setting display i4, a user can transmits a message to the communicator 113 in the aerosol generator 100 to notify it of the message. The massage may comprise text information including the Japanese language or the English language (alphabets), and/or image information comprising pictorial symbols, stamps, and so on. Further, the mode of notifiation to the aerosol generator 100 may be set freely. In the example in Fig. 8A, a user has inputted Japanese characters, which together represent the expression "test," and is attempting to inform the communicator 113 in the aerosol generator 100 of the above expression.

As explained above, the operation setting of the aerosol generator 100 that the user can request is associated with at least one of functions of (i2) profile setting of heating operation in the aerosol generator 100, (i3) locking/unlocking operation of the aerosol generator 100, and (i4) notification of information inputted by a user.

In addition, like the case of the example in Fig. 8A, it may be possible to include, in the flavor adjustment display i2, the device lock setting display i3, and the message notification setting display i4, reflecting buttons bt1-bt3 for requesting operation setting, respectively. That is, in terms of the respective functions, a user can make respective requests for operation setting of the aerosol generator 100, independently.

In the above case, in terms of the respective functions, respective display statuses st1-st3 of respective setting requests may be displayed independently. In the example in Fig. 8A, every one of the display statuses st1-st3 represents the status "Waiting for Reflection." For example, in the flavor adjustment display i2, as a result that a user has selected the "Strong" profile and pressed (i.e., touched) the "Reflecting Adjustment" button bt1, the display status st1 corresponding thereto has been set to show "Waiting for Reflection." Further, in the device lock setting display i3, as a result that a user has selected the "Unlocking" button and pressed the "Reflecting Lock Setting" button bt2, the display status st1 corresponding thereto has been set to show "Waiting for Reflection." Similarly, in the message notification setting display i4, as a result that a user has inputted a message and pressed the "Reflecting Notification" button bt3, the display status st1 corresponding thereto has been set to show "Waiting for Reflection."

That is, as a result that the respective buttons bt1-bt3 have been pressed by the user, the respective display statuses st1-st3 are being set to show "Waiting for Reflection," independently (step S23). In the above case, each of the display status st1-st3 will be changed to show the status "Reflection Completed" (step S26), right after receiving of a completion confirmation after actual reflecting of the operation setting requested by the user in the aerosol generator 100; and the state is changed to allow accepting of a next setting request (step S27).

In a different construction, for example, in the case that the "Reflecting Adjustment" button bt1 is pressed and the display status st1 is set to show "Waiting for Reflection" in the flavor adjustment display i2, the display states st2 and st3 relating to other operation setting i3 and i4 may also be set to show "Waiting for Reflection" in conjunction with the above operation (step S23). The above construction has been designed for preventing accepting of plural setting requests from a user during a same period of time, since there are restrictions in data communication to the aerosol generator 100, for example, there is a restriction that the number of times of data communication is limited to at most 4 times per day as explained above. In this regard, since a user is disallowed to further request operation setting during the period of time when the display status is "Waiting for Reflection" (step S24), inputting of plural setting requests within a short period of time by the user can be prevented. In the above case, in relation to the flavor adjustment display i2, right after receiving of the completion confirmation after reflecting of the operation setting with respect to the "Strong" profile requested by the user in the aerosol generator 100, all the display statuses st1-st3 are changed at the same time to make each of them show the status "Reflection Completed" (step S26). Thereafter, the state is set to allow accepting of a next setting request (step S27). In a different construction, in the case that the number of times of data communication exceeds the above limited number of times of data communication defined as a restriction (at most 4 times per day), the state that does not allow accepting of a next setting request may be maintained until a next day comes and the restriction is removed.

On the other hand, in the example in Fig. 8B, combinations of the reflecting buttons bt1-bt3 and the display statues st1-st3 are integrated into graphical switches sw1-sw3, respectively. With respect to the flavor adjustment display i2, the example in Fig. 8B is that showing a scene after the side of "ON" of the sw1 is pressed by a user for requesting operation setting of the profile to set it to "Strong." As a result that the switch sw1 shows an "ON" state and the display is deactivated, the state that the display status is "Waiting for Reflection" is presented to the user (step S23), and, at the same time, in this example, further setting of the profile is disallowed (step S24). Further, in the example in Fig. 8B, the waiting time t1 is displayed in a position on a side of the switch sw1 as restriction information, wherein the restriction information displayed herein is "7 seconds until reflection" (step S25).

Right after receiving of the completion confirmation after reflecting of the operation setting with respect to the "Strong" profile in the aerosol generator 100, the switch sw1 shows an "OFF" state and the display is activated again. As a result, the state that the display status has been changed to "Reflection Completed" is presented to the user (step S26), and the state is set to allow accepting of a next setting request (step S27).

In the example in Fig. 8B, the switch sw2 for the device lock setting display i3 and the switch sw3 for the message notification setting display i4 are activated independent of the switch sw1 for the flavor adjustment display i2. That is, each of the switches sw2 and sw3 can accept a setting request independent of the switch sw1.

### « 3. Modification Examples »

It has been explained, in relation to the restricted data communication, in the above description relating to the embodiments that the communication manager 320 in the server 300 calculates, based on information of the restriction, the waiting time until the time of removal of the restriction, (steps S321 and S323). Instead of the above construction, in a modification example, the communication manager 320 may determine the waiting time, based at least on the history of past waiting time that relates to a user and has been recorded in the server 300.

For example, the maximum waiting time, an average waiting time, or the like may be determined as the waiting time, by using information of past waiting time relating to a user of the user terminal 200. Further, the waiting time may be determined, based on the maximum waiting time, an average waiting time, or the like and by using a predetermined mathematical formula.

In a different construction, the waiting time until the time of removal of the restriction may be estimated by preparing an inference engine and using it, wherein the inference engine is prepared by making it learn correlation between the waiting time and the number of times and timing of occurrence of setting requests in a day by making it use the histories of past waiting time relating to all users registered with the server 300. In an example, the above inference engine may be constructed by performing supervised machine learning, wherein the history data of the number of times and timing of occurrence of setting requests in a day is used as the feature quantity, and the history data of the waiting time corresponding to the above feature quantity is used as label data.

### « 4. Other Embodiments »

In the above explanation, information processing terminals, information processing methods, and information processing systems relating to some embodiments have been explained with reference to the figures. It will be understood that the present disclosure can be implemented as a program for making a processor operate the information processing terminal and/or execute the information processing method when the program is executed by the processor, and/or a computer-readable recording medium which stores the program.

In the above description, embodiments of the present disclosure have been explained together with their modification examples and application modes; and, in this regard, it should be understood that they are mere examples, and they are not those limiting the scope of the present disclosure. It should be understood that change, addition, modification, and so on with respect to the embodiments can be performed appropriately, without departing from the gist and the scope of the present disclosure. The scope of the present disclosure should not be limited by any of the above-explained embodiments, and should be defined by the claims and equivalents thereof only.

Further, the constructions such as those shown below are constructions within the scope of the technique of the present invention.
(1) An information processing method comprising steps for:
   accepting a first setting request relating to operation setting of an inhaler;
   transmitting the first setting request, that is directed to the inhaler, to a server;
   changing, in response to transmission of the first setting request, a display status relating to the inhaler to that representing a waiting-for-reflection state;
   receiving, from the inhaler via the server, a completion confirmation that represents completion of the operation setting in the inhaler; and
   changing, in response to the completion confirmation, the display status from that representing the waiting-for-reflection state to that representing a reflection-completed state.
(2) The information processing method recited in above item (1) further comprising a step for
   disallowing accepting of a second setting request relating to operation setting of the inhaler, during a period of time when the display status is that representing the waiting-for-reflection state.
(3) The information processing method recited in above item (2) further comprising a step for
   allowing accepting of the second setting request, in response to completion of changing of the display status from that representing the waiting-for-reflection state to that representing the reflection-completed state.
(4) The information processing method recited in any one of above items (1)-(3), wherein
   a predetermined restriction is related to communication with the inhaler, in a wireless network to which the inhaler is connected,
   the method further comprises a step for receiving, from the server after the first setting request is transmitted to the server, restriction information determined in the server, and
   the restriction information comprises waiting time until the time when the predetermined restriction is removed.
(5) The information processing method recited in any one of above items (1)-(4), wherein
   the operation setting of the inhaler is related to at least one of
   a function relating to a profile of heating operation in the inhaler,
   a function of locking operation of the inhaler, and
   a function of displaying of information inputted by a user.
(6) The information processing method recited in any one of above items (1)-(5), wherein
   the operation setting is related to plural functions that the inhaler has, and
   each of the plural functions is related to the display status.
(7) The information processing method recited in item (6), wherein
   the plural functions comprises a first function and a second function,
   the first setting request is related to the first function, and
   in response to transmission of the first setting request, the display statuses relating to the inhaler, that relate to the first function and the second function, are changed to represent the waiting-for-reflection states, respectively.
(8) An information processing terminal comprising:
   a reception unit for accepting a first setting request relating to operation setting of an inhaler;
   a transmitter for transmitting the first setting request, that is directed to the inhaler, to a server;
   a receiver for receiving, from the inhaler via the server, a completion confirmation that represents completion of the operation setting in the inhaler; and
   a status transition unit for
      changing, in response to transmission of the first setting request, a display status relating to the inhaler to that representing a waiting-for-reflection state, and
      changing, in response to a completion confirmation from the inhaler, the display status from that representing the waiting-for-reflection state to that representing a reflection-completed state.
(9) The information processing terminal recited in above item (8) further comprising
   a reception controller which disallows accepting of a second setting request relating to operation setting of the inhaler, during a period of time when the display status is that representing the waiting-for-reflection state.
(10) The information processing terminal recited in above item (9), wherein
   the reception controller is further constructed to allow accepting of the second setting request, in response to completion of changing of the display status from that representing the waiting-for-reflection state to that representing the reflection-completed state.
(11) The information processing terminal recited in any one of above items (8)-(10), wherein
   a predetermined restriction is related to communication with the inhaler, in a wireless network to which the inhaler is connected,
   the information processing terminal is constructed in such a manner that the receiver receives, from the server after the first setting request is transmitted to the server from the transmitter, restriction information determined in the server, and
   the restriction information comprises waiting time until the time when the predetermined restriction is removed.
(12) An information processing system comprising an information processing terminal, a server, and an inhaler, wherein:
   the information processing terminal comprises
      a reception unit for accepting a setting request relating to operation setting of the inhaler, and
      a first communicator for transmitting the setting request, that is directed to the inhaler, to a serve, and receiving, from the server, a completion confirmation that represents completion of the operation setting in the inhaler;
   the server comprises
      a second communicator for transmitting, to the inhaler via an external server, the setting request received from the information processing terminal, and transmitting, to the information processing terminal, the completion confirmation received from the inhaler via the external server, and
      a communication manager for managing, based on a predetermined restriction defined with respect to communication between the external server and the inhaler, transmission of the setting request by the second communicator; and
   the inhaler comprises
      a third communicator for transmitting, to the external server, the completion confirmation relating to the setting request received from the server via the external server, and
      a controller for performing, in response to the setting request, the operation setting in the inhaler.
(13) The information processing system recited in above item (12), wherein
   the information processing terminal further comprises a status transition unit for
      changing, in response to transmission of the setting request, a display status relating to the inhaler to that representing a waiting-for-reflection state, and
      changing, in response to the completion confirmation, the display status from that representing the waiting-for-reflection state to that representing a reflection-completed state, and
   the display status is displayed by the information processing terminal.
(14) The information processing system recited in above item (12) or (13), wherein
   the communication manager in the server is constructed to
   judge, in response to reception of the setting request by the second communicator, whether the communication is restricted, and
   hold transmission of the setting request to the inhaler, if the communication is restricted.
(15) The information processing system recited in any one of above items (12)-(14), wherein
   the communication manager in the server is constructed to determine restriction information that is based on the predetermined restriction,
   the second communicator in the server is constructed to transmit the restriction information to the information processing terminal, and
   the restriction information comprises waiting time until the time when the restriction relating to the communication is removed.
(16) The information processing system recited in above item (15), wherein
   the waiting time is determined based at least on history of past waiting time that relates to the user and has been recorded in the server.
(17) The information processing system recited in any one of above items (12)-(16), wherein
   the predetermined restriction relating to the communication comprises a restriction such that transmission of the setting request to the inhaler is allowed to be performed only a predetermined number of times within a predetermined period of time.
(18) The information processing system recited in any one of above items (12)-(17), wherein
   the predetermined restriction relating to the communication comprises a restriction such that transmission of the setting request to the inhaler is allowed to be performed only within a predetermined allowed period of time starting at each predetermined time interval.
(19) The information processing system recited in above item (17) or (18), wherein
   the third communicator in the inhaler is further constructed to
   periodically transmit a communication request to the external server, and
   receive the setting request as a response to the communication request.
(20) The information processing system recited in any one of above items (12)-(19), wherein
   the wireless network, to which the inhaler is connected, conforms to an LPWA (Low Power Wide Area) wireless communication standard.
(21) A computer-readable recording medium recording a computer program which makes a server, when the program is executed by a processor in the server, perform operation for:
   periodically receiving a communication request from an inhaler via an external server;
   receiving, from an information processing terminal, a setting request relating to operation setting of the inhaler;
   holding, based on a predetermined restriction relating to data communication in a wireless network to which the inhaler is connected, transmission of the setting request;
   transferring the setting request, as a response to the communication request, to the inhaler via the external server after the predetermined restriction is removed; and
   transferring, to the information processing terminal, a completion conformation that has been received from the inhaler via the external server and represents completion of the operation setting in the inhaler.
(22) The computer-readable recording medium recited in above item (21) which further makes a server perform operation for:
   determining, based on the predetermined restriction, restriction information relating to the setting request; and
   transmitting the determined restriction information to the information processing terminal; wherein
   the restriction information comprises waiting time until the time when the predetermined restriction is removed.
(23) The computer-readable recording medium recited in above item (21) or (22), wherein
   the predetermined restriction comprises a restriction such that transmission of the setting request to the inhaler is allowed to be performed only a predetermined number of times within a predetermined period of time.
(24) The computer-readable recording medium recited in any one of above items (21)-(23), wherein
   the predetermined restriction comprises a restriction such that transmission of the setting request to the inhaler is allowed to be performed only within a predetermined allowed period of time starting at each predetermined time interval.
(25) A computer program which makes a computer perform the information processing method recited in any one of above items (1)-(7).

### REFERENCE SIGNS LIST

100, 100A, 100B ... Aerosol generator: 113 ... Notifier: 115 ... Communicator: 116 ... Controller: 200 ... User terminal: 205 ... Application: 210 ... Communicator: 212 ... Transmitter: 214 ... Receiver: 220 ... Reception unit: 230 ... Status display: 240 ... Status transition unit: 250 ... Reception controller: 300 ... Server: 310 ... Communicator: 320 ... Communication manager: 400 ... External server: 500, 600, 700 ... Network

## Claims

1. An information processing method comprising steps for:
accepting a first setting request relating to operation setting of an inhaler;
transmitting the first setting request, that is directed to the inhaler, to a server;
changing, in response to transmission of the first setting request, a display status relating to the inhaler to that representing a waiting-for-reflection state;
receiving, from the inhaler via the server, a completion confirmation that represents completion of the operation setting in the inhaler; and
changing, in response to the completion confirmation, the display status from that representing the waiting-for-reflection state to that representing a reflection-completed state.

2. The information processing method as recited in Claim 1 further comprising a step for
disallowing accepting of a second setting request relating to operation setting of the inhaler, during a period of time when the display status is that representing the waiting-for-reflection state.

3. The information processing method as recited in Claim 2 further comprising a step for
allowing accepting of the second setting request, in response to completion of changing of the display status from that representing the waiting-for-reflection state to that representing the reflection-completed state.

4. The information processing method as recited in any one of Claims 1-3, wherein
a predetermined restriction is related to communication with the inhaler, in a wireless network to which the inhaler is connected,
the method further comprises a step for receiving, from the server after the first setting request is transmitted to the server, restriction information determined in the server, and
the restriction information comprises waiting time until the time when the predetermined restriction is removed.

5. The information processing method as recited in any one of Claims 1-4, wherein
the operation setting of the inhaler is related to at least one of
a function relating to a profile of heating operation in the inhaler,
a function of locking operation of the inhaler, and
a function of informing of information inputted by a user.

6. The information processing method as recited in any one of Claims 1-5, wherein
the operation setting is related to plural functions that the inhaler has, and
each of the plural functions is related to the display status.

7. The information processing method as recited in Claim 6, wherein
the plural functions comprises a first function and a second function,
the first setting request is related to the first function, and
in response to transmission of the first setting request, the display statuses relating to the inhaler, that relate to the first function and the second function, are changed to represent the waiting-for-reflection states, respectively.

8. An information processing terminal comprising:
a reception unit for accepting a first setting request relating to operation setting of an inhaler;
a transmitter for transmitting the first setting request, that is directed to the inhaler, to a server;
a receiver for receiving, from the inhaler via the server, a completion confirmation that represents completion of the operation setting in the inhaler; and
a status transition unit for
changing, in response to transmission of the first setting request, a display status relating to the inhaler to that representing a waiting-for-reflection state, and
changing, in response to a completion confirmation from the inhaler, the display status from that representing the waiting-for-reflection state to that representing a reflection-completed state.

9. The information processing terminal as recited in Claim 8 further comprising
a reception controller which disallows accepting of a second setting request relating to operation setting of the inhaler, during a period of time when the display status is that representing the waiting-for-reflection state.

10. The information processing terminal as recited in Claim 9, wherein
the reception controller is further constructed to allow accepting of the second setting request, in response to completion of changing of the display status from that representing the waiting-for-reflection state to that representing the reflection-completed state.

11. The information processing terminal as recited in any one of Claims 8-10, wherein
a predetermined restriction is related to communication with the inhaler, in a wireless network to which the inhaler is connected,
the information processing terminal is constructed in such a manner that the receiver receives, from the server after the first setting request is transmitted to the server from the transmitter, restriction information determined in the server, and
the restriction information comprises waiting time until the time when the predetermined restriction is removed.

12. An information processing system comprising an information processing terminal, a server, and an inhaler, wherein:
the information processing terminal comprises
a reception unit for accepting a setting request relating to operation setting of the inhaler, and
a first communicator for transmitting the setting request, that is directed to the inhaler, to a serve, and receiving, from the server, a completion confirmation that represents completion of the operation setting in the inhaler;
the server comprises
a second communicator for transmitting, to the inhaler via an external server, the setting request received from the information processing terminal, and transmitting, to the information processing terminal, the completion confirmation received from the inhaler via the external server, and
a communication manager for managing, based on a predetermined restriction defined with respect to communication between the external server and the inhaler, transmission of the setting request by the second communicator; and
the inhaler comprises
a third communicator for transmitting, to the external server, the completion confirmation relating to the setting request received from the server via the external server, and
a controller for performing, in response to the setting request, the operation setting in the inhaler.

13. The information processing system as recited in Claim 12, wherein
the information processing terminal further comprises a status transition unit for
changing, in response to transmission of the setting request, a display status relating to the inhaler to that representing a waiting-for-reflection state, and
changing, in response to the completion confirmation, the display status from that representing the waiting-for-reflection state to that representing a reflection-completed state, and
the display status is displayed by the information processing terminal.

14. The information processing system as recited in Claim 12 or 13, wherein
the communication manager in the server is constructed to
judge, in response to reception of the setting request by the second communicator, whether the communication is restricted, and
hold transmission of the setting request to the inhaler, if the communication is restricted.

15. The information processing system as recited in any one of Claims 12-14, wherein
the communication manager in the server is constructed to determine restriction information that is based on the predetermined restriction,
the second communicator in the server is constructed to transmit the restriction information to the information processing terminal, and
the restriction information comprises waiting time until the time when the restriction relating to the communication is removed.

16. The information processing system as recited in Claim 15, wherein
the waiting time is determined based at least on history of past waiting time that relates to the user and has been recorded in the server.

17. The information processing system as recited in any one of Claims 12-16, wherein
the predetermined restriction relating to the communication comprises a restriction such that transmission of the setting request to the inhaler is allowed to be performed only a predetermined number of times within a predetermined period of time.

18. The information processing system as recited in any one of Claims 12-17, wherein
the predetermined restriction relating to the communication comprises a restriction such that transmission of the setting request to the inhaler is allowed to be performed only within a predetermined allowed period of time starting at each predetermined time interval.

19. The information processing system as recited in Claim 17 or 18, wherein
the third communicator in the inhaler is further constructed to
periodically transmit a communication request to the external server, and
receive the setting request as a response to the communication request.

20. The information processing system as recited in any one of Claims 12-19, wherein
the wireless network, to which the inhaler is connected, conforms to an LPWA (Low Power Wide Area) wireless communication standard.
